# EUROPEAN PATENT APPLICATION

(11) **EP 2 193 743 A1**
(43) Date of publication of application: **09.06.2010**
(21) Application number: 09252706.8
(22) Date of filing: 01.12.2009
(51) Int. Cl.: A61B 1/005

(54) **Endoscope flexible portion and endoscope**

(30) Priority: 04.12.2008 JP 2008309331
(71) Applicant: FUJIFILM Corporation, Minato-ku Tokyo (JP)
(72) Inventor: Fukunaga, Toshiaki, Ashigarakami-gun Kanagawa (JP)
(74) Representative: Stevens, Jason Paul

(57) **Abstract**

An endoscope flexible portion (30) includes a spiral tube (32) that is formed by spirally winding a strip-shaped member, a mesh tube (34) that is disposed on the outer periphery of the spiral tube (32) and is formed by braiding together fine wires (34A), an operation-use angle wire (38) that is disposed inside the spiral tube (32) and is formed by bundling together metal strands (64) into a stranded wire (62) in order to operate a curving portion disposed on a distal end side of the endoscope, and an outer sheath member (36) that covers the outer periphery of the mesh tube (34). A coating layer (42) formed of poly-para-xylylene resin is formed on the outer peripheral surface of at least one of the spiral tube (32), the mesh tube (34) or the operation-use angle wire (38). The invention also extends to an endoscope using such a flexible portion.

## Description

The present invention relates to a tubular endoscope flexible (soft) portion and to an endoscope equipped with this endoscope flexible portion.

Medical endoscopes are used to observe organs and the like by inserting an elongate insertion portion inside a body cavity of a patient and to perform various treatments and procedures using accessories inserted inside an accessory insertion channel in the endoscope. For this reason, when an endoscope that has been used once is to be reused in another patient, it is necessary to disinfect and sterilize the endoscope after the end of examination and treatment in order to prevent infection between patients via the endoscope. For disinfection and sterilization, there are methods that use disinfectant solutions, ethylene oxide gas, formalin gas, hydrogen peroxide gas, plasma, ozone, or autoclaving, which is sterilization that uses high-temperature high-pressure steam.

The autoclaving method, which sterilizes endoscopes with high-temperature high-pressure steam, is a sterilization method that has conventionally become widespread. This method has many advantages, such as the reliability of he sterilization effect is high, there is no residual toxicity, and running costs are inexpensive. However, as typical conditions when sterilizing endoscopes with high-temperature high-pressure steam, it is prescribed in ANSI/AAMI ST 37-1992 approved by the American National Standards Institute (ANSI) and published by the Association for the Advancement of Medical Instrumentation (AAMI) that the sterilization process should be at 132° C for 4 minutes in the pre-vacuum type and that the sterilization process should be at 132° C for 10 minutes in the gravity type, and under such an environment, damage to medical equipment can become extremely large.

The flexible portion (flexible tube) that forms the insertion portion of an endoscope is, for example, equipped with a spiral tube that is formed by spirally winding a strip-shaped member in a constant diameter and a mesh tube that is formed by braiding together fine wires such as a stainless steel wires on the outer periphery of this spiral tube. Because of this mesh tube, strength with respect to expansion/contraction and torsion is increased, and strength with respect to crushing of the insertion portion of the endoscope is increased. Moreover, an outer sheath layer comprising a thermoplastic elastomer or the like covers the outer periphery of this mesh tube of the flexible portion (flexible tube) of the endoscope. In the flexible portion (flexible tube) of such an endoscope, when a disinfection and sterilization treatment by autoclaving or using oxidizing chemicals is repeatedly performed, the fine wires such as stainless steel wires can be deteriorated by oxidization and rust, and the strength of the flexible, portion (flexible tube) of the endoscope can thus drop.

Japanese Patent Application Laid-Open Publication (JP-A) No. 2006-198234 discloses a configuration where a silica coating layer is formed on the outer surfaces of the fine wires of the mesh tube. In this configuration, the barrier property can be raised by forming the silica coating layer, but there is the potential for the silica coating layer to come off by repeating operation such as torsion.

In the case of an endoscope that uses, as an angle wire for operating the curving portion of the endoscope flexible portion, a wire that is formed by bundling together metal strands of stainless steel or the like into a stranded wire, steam entering the inside of the endoscope may cause the angle wire to deteriorate when a disinfection and sterilization treatment resulting from autoclaving is repeatedly performed. Further, there is the potential for water to remain in the gaps between the strands of the wire because of this steam, such that the wire rusts and causes curvature defects.

JP-A No. 2001-46330 discloses a configuration where the surface of the angle wire is coated with a rust-preventing agent that has high-temperature high-pressure steam resistance. However, in this configuration, it is difficult to evenly coat the surface of the angle wire with the rust-preventing agent, water resistance, the gas barrier property and heat resistance are not sufficient, and it is not always the case that resistance with respect to a disinfection and sterilization treatment by autoclaving or the like is sufficient.

In consideration of the above-described circumstances, at least the preferred embodiments of the present invention provide an endoscope flexible portion and an endoscope that can control oxidation and deterioration of the mesh tube, the angle wire and the like at the time of sterilization with high-temperature high-pressure steam.

According to a first aspect of the invention, there is provided an endoscope flexible portion including: a spiral tube that is formed by spirally winding a strip-shaped member and has a hollow portion; a mesh tube that is disposed on the outer periphery of the spiral tube and is formed by braiding together fine wires; an operation-use angle wire that is disposed inside the spiral tube and is formed by bundling together metal strands into a stranded wire in order to operate a curving portion disposed on a distal end side of the endoscope; and an outer sheath member that covers the outer periphery of the mesh tube, wherein a coating layer comprising poly-para-xylylene resin is formed on the outer peripheral surface of at least one of the spiral tube, the mesh tube or the operation-use angle wire.

By forming the coating layer formed of poly-para-xylylene resin on the outer peripheral surface of at least one of the spiral tube and the mesh tube, oxidation and deterioration of the spiral tube and the mesh tube during sterilization with high-temperature high-pressure steam are controlled. Thus, deformation of the endoscope flexible portion (particularly the insertion portion) can be controlled. Further, by forming the coating layer formed of poly-para-xylylene resin on the outer peripheral surface of the operation-use angle wire that is formed by bundling together metal strands into a stranded wire, adhesion between the poly-para-xylylene resin and the metal strands is high, and oxidation and deterioration of the metal strands can be controlled at the time of sterilization with high-temperature high-pressure steam.

In a preferred form, the mesh tube is formed by forming the coating layer comprising poly-para-xylylene resin on the fine wires and braiding together those fine wires.

With this arrangement, oxidation and deterioration of the mesh tube at the time of sterilization with high-temperature high-pressure steam can be further controlled.

In a further preferred form, the operation-use angle wire may be formed by forming the coating layer comprising poly-para-xylylene resin on the metal strands and bundling together those metal strands into a stranded wire.

In this configuration, the poly-para-xylylene resin can coat the entire outer peripheral surfaces of the metal strands, and adhesion between the poly-para-xylylene resin and the metal strands is high, so oxidation and deterioration of the metal strands can be further controlled at the time of sterilization with high-temperature high-pressure steam.

It is further preferred for the operation-use angle wire on which the coating layer comprising poly-para-xylylene resin has been formed to be coated with a lubricant that reduces the coefficient of friction more than the coating layer comprising poly-para-xylylene resin.

According to this configuration, deterioration of the spiral tube and the mesh tube resulting from friction of the operation-use angle wire can be controlled.

The invention also extends to an endoscope whose elongate insertion portion which is inserted inside a body cavity of a patient is formed by an endoscope flexible portion as described above.

By forming the coating layer formed of poly-para-xylylene resin on the outer peripheral surface of at least one of the spiral tube and the mesh tube, oxidation and deterioration of the spiral tube and the mesh tube at the time of sterilization with high-temperature high-pressure steam are controlled. Thus, deformation of the endoscope flexible portion (particularly the insertion portion) can be controlled. Further, by forming the coating layer of poly-para-xylylene resin on the outer peripheral surface of the operation-use angle wire that is formed by bundling together metal strands into a stranded wire, adhesion between the poly-para-xylylene resin and the metal strands is high, so oxidation and deterioration of the metal strands can be controlled at the time of sterilization with high-temperature high-pressure steam.

As described above, in these aspects, oxidation and deterioration of at least one of the mesh tube, the spiral tube and the operation-use angle wire can be controlled at the time of sterilization with high-temperature high-pressure steam.

Preferred embodiments of the invention will now be described by way of example only and with reference to the accompanying drawings, in which:
FIG. 1 is a general configuration view showing an endoscope that uses an endoscope flexible portion in a first embodiment;
FIG. 2 is a partially exploded side view showing the configuration of the endoscope flexible portion in the first embodiment;
FIG. 3 is a partially exploded perspective view showing the configuration of the endoscope flexible portion in the first embodiment;
FIG. 4 is an enlarged perspective view showing a part of a mesh tube that is used in the endoscope flexible portion in the first embodiment;
FIG. 5 is an enlarged perspective sectional view showing part of the mesh tube that is used in the endoscope flexible portion in the first embodiment;
FIG. 6 is a sectional view showing a spiral tube that is used in an endoscope flexible portion in a second embodiment;
FIG. 7 is a sectional view showing an operation-use angle wire that is used in an endoscope flexible portion in a third embodiment; and
FIG. 8 is a perspective sectional view showing a metal strand of the operation-use angle wire that is used in the endoscope flexible portion in the third embodiment.

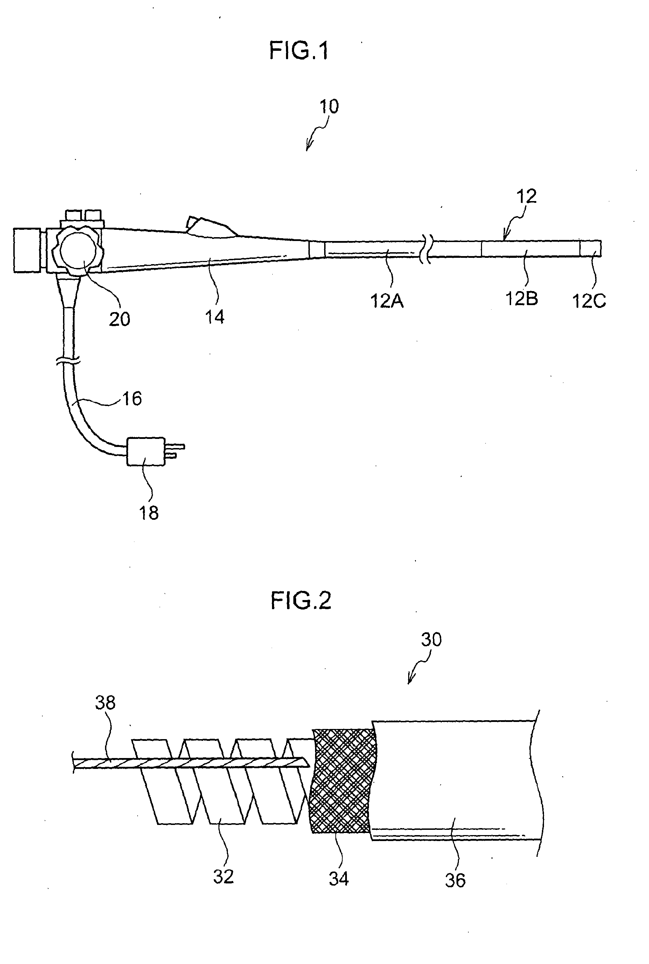

In FIG. 1, there is shown the overall configuration of an endoscope 10 pertaining to a first embodiment. As shown in FIG. 1, the endoscope 10 has an elongate insertion portion 12 that is inserted into a body cavity of a patient, and an operation portion 14 is disposed consecutively with the proximal end portion of the insertion portion 12. An elongate light guide flexible portion 16 that is detachably connected to a light source device (not shown) is coupled to the operation portion 14. A connector 18 having a terminal that is connected to the light source device is disposed on the distal end of the light guide flexible portion 16. Further, an operation knob 20 for operating the insertion portion 12 is disposed on the operation portion 14.

The insertion portion 12 has a flexible tube portion 12A serving as an endoscope flexible portion that forms the majority of the length portion of the insertion portion 12 in the longitudinal direction (axial direction) from the portion disposed consecutively with the operation portion 14, an angle portion 12B that is disposed consecutively with the longitudinal direction distal end side of this flexible tube portion 12A, and a distal end portion 12C that is disposed consecutively with the longitudinal direction distal end side of the angle portion 12B and has a built-in objective optical system and the like. The angle portion 12B is configured such that it can be remotely bent by rotating the operation knob 20 disposed on the insertion portion 12. Further, the light guide flexible portion 16 also has substantially the same structure as that of the flexible tube portion 12A of the insertion portion 12.

The flexible tube portion 12A has a length such that the distal end portion 12C can reach the inside of a predetermined observation target area, and the flexible tube portion 12A is set to a length where it can be kept away from the patient or the like to an extent that it does not disrupt an operator gripping and operating the operation portion 14. It is necessary for the flexible tube portion 12A to be given flexibility across substantially its entire length, and the flexible tube portion 12A has a structure where particularly the part that is inserted inside a body cavity of a patient has sufficient flexibility. Further, particularly at the portion where the flexible tube portion 12A is disposed consecutively with the operation portion 14, some rigidity is necessary with respect to bending in order to obtain a push-in thrust force when inserting the insertion portion 12 inside a body cavity. It is preferred that the portion where particularly the flexible tube portion 12A is disposed consecutively with the angle portion 12B has sufficient flexibility in order to allow it to follow the curved shape to a certain extent when the angle portion 12B curves.

The flexible tube portion 12A houses, inside a tubular portion, a light guide, an image guide (a signal cable in the case of an electronic endoscope), an accessory insertion channel and air/water tubes that are not shown.

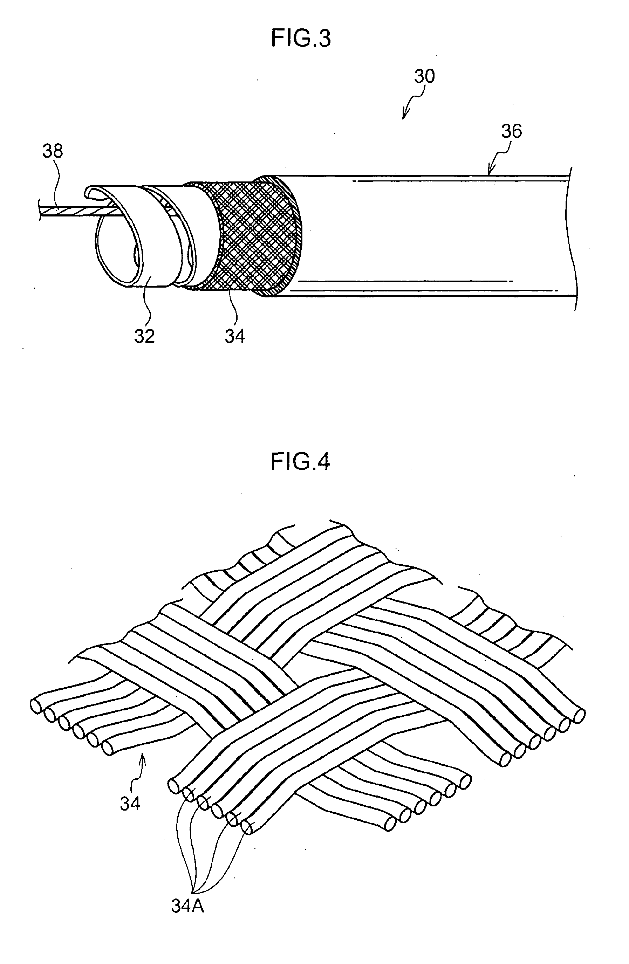

FIG. 2 shows an endoscope flexible portion 30 that forms the tubular portion of the flexible tube portion 12A in a partial cutaway side view. FIG. 3 shows the endoscope flexible portion 30 in a partial cutaway perspective view. As shown in FIG. 2 and FIG. 3, the endoscope flexible portion 30 has a spiral tube 32 that is formed by spirally winding a strip-shaped member made of metal, a mesh tube 34 that is formed by braiding together fine wires 34A on the outer periphery of this spiral tube 32, and an outer sheath member 36 that is formed on the outer periphery of this mesh tube 34. An operation-use angle wire 38 that is formed by bundling together metal strands into a stranded wire in order to operate a curving portion disposed on a distal end side is disposed inside the spiral tube 32.

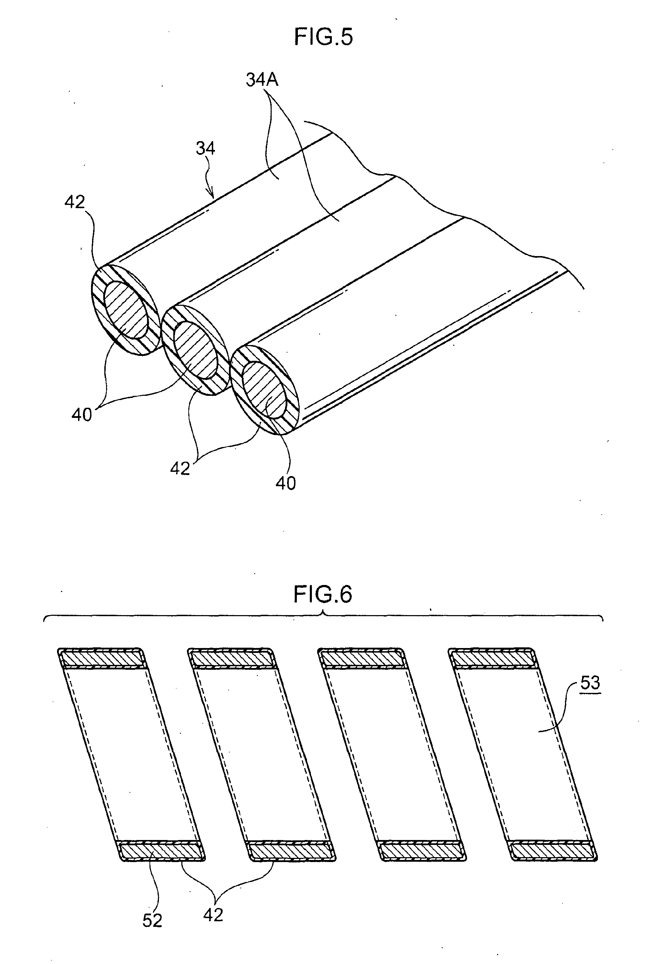

As shown in FIG. 4 and FIG. 5, the mesh tube 34 is formed by forming a coating layer 42 formed of poly-para-xylylene resin on the metal strands 40 that form the fine wires 34A and braiding together those fine wires 34A on which the coating layer 42A has been formed. It is preferred that the thickness of the coating layer 42 of poly-para-xylylene resin is equal to or greater than 5 µm from the standpoint of being damp-proof.

Next, a method of creating the endoscope flexible portion 30 will be described.

The metal strands 40 that form the mesh tube 34 are placed inside a chamber in a chemical vapour deposition device, the chamber is depressurized, vaporized di-para-xylylene is thereafter fed through a heating tube into the inside of the chamber, and the metal strands 40 are left in that atmosphere for a predetermined amount of time. That is, the coating layer 42 of poly-para-xylylene resin is formed by the chemical vapour deposition of poly-para-xylylene, for example. Examples of poly-para-xylylene resin include poly-monochloro-para-xylylene, poly-para-xylylene, poly-dichloro-para-xylylene and fluorinated poly-para-xylylene, and one whose properties are suitable is selected or combined to make a coating agent. The coating thickness (the thickness of the coating layer 42) can be adjusted by varying the amount of time spent in the resin atmosphere. It is preferred that the thickness of the coating layer 42 comprising poly-para-xylylene resin is about 5 to 80 µm. When the thickness of the coating layer 42 is smaller than 5 µm, it is difficult to ensure the desired bending rigidity. Further, when the thickness of the coating layer 42 is larger than 80 µm, it is difficult to maintain the flexibility of the endoscope flexible portion.

After the coating layer 42 of poly-para-xylylene resin has been formed on the metal strands 40, those metal strands 40 are braided together to form the mesh tube 34.

Next, the operation and effects of the endoscope flexible portion 30 will be described.

The mesh tube 34 of the endoscope flexible portion 30 is formed by forming the coating layer 42 of poly-para-xylylene resin on the metal strands 40 that form the fine wires 34A and braiding together those fine wires 34A on which the coating layer 42 has been formed. By forming the coating layer 42 of poly-para-xylylene resin on the outer peripheral surface of the mesh tube 34, oxidation and deterioration of the mesh tube 34 at the time of sterilization with high-temperature high-pressure steam can be controlled. Thus, deformation of the endoscope flexible portion 30 (particularly the insertion portion) can be controlled.

Next, an endoscope flexible portion pertaining to a second embodiment will be described. It will be noted that the same reference numerals will be given to members that are the same as those in the first embodiment and that redundant description will be omitted.

As shown in FIG. 6, a spiral tube 52 that configures the endoscope flexible portion has a hollow portion 53 that is formed by spirally winding a strip-shaped member made of metal, and the coating layer 42 formed of poly-para-xylylene resin is formed on the outer peripheral surface of the spiral tube 52.

The endoscope flexible portion equipped with this spiral tube 52 is created as follows.

The spiral tube 52 is placed inside a chamber in a chemical vapour deposition device, the chamber is depressurized, vaporized di-para-xylylene is thereafter fed through a heating tube into the inside of the chamber, and the spiral tube 52 is left in that atmosphere for a predetermined amount of time. That is, the coating layer 42 of poly-para-xylylene resin is formed by the chemical vapour deposition of poly-para-xylylene, for example. The coating layer 42 of poly-para-xylylene resin discussed above is formed on the outer peripheral surface of the spiral tube 52 that is formed by spirally winding a strip-shaped member made of metal.

In the endoscope flexible portion having the spiral tube 52, by forming the coating layer 42 of poly-para-xylylene resin on the outer peripheral surface of the spiral tube 52, oxidation and deterioration of the spiral tube 52 at the time of sterilization with high-temperature high-pressure steam can be controlled. Thus, deformation of the endoscope flexible portion (particularly the insertion portion) can be controlled.

Next, an endoscope flexible portion pertaining to a third embodiment will be described. It will be noted that the same reference numerals will be given to members that are the same as those in the first and second embodiments and that redundant description will be omitted.

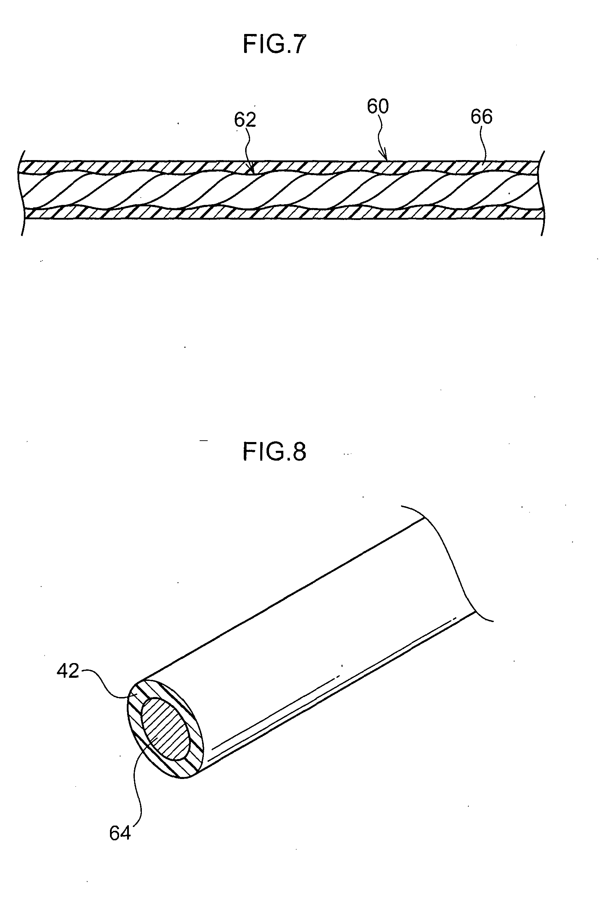

As shown in FIG. 7, an operation-use angle wire 60 that is used in the endoscope flexible portion has a wire core portion 62. The wire core portion 62 is, as shown in FIG. 8, formed by forming the coating layer 42 of poly-para-xylylene resin on metal strands 64 and bundling together those metal strands 64 on which the coating layer 42 has been formed into a stranded wire. As shown in FIG. 7, on the outer peripheral surface of the wire core portion 62, there is formed a lubricant coating layer 66 formed of a lubricant that reduces the coefficient of friction more than the coating layer 42 formed of poly-para-xylylene resin. As the lubricant coating layer 66, there is used at least one type of lubricant selected from the group including, for example, MoS₂ powder, BN powder, carbon graphite powder, rice bran ceramic, fluorinated oil, fluorinated grease and polytetrafluoroethylene powder.

The endoscope flexible portion equipped with this operation-use angle wire 60 is created as follows.

The metal strands 64 that become the material of the operation-use angle wire 60 are placed inside a chamber in a chemical vapour deposition device, the chamber is depressurized, vaporized di-para-xylylene is thereafter fed through a heating tube into the inside of the chamber, and the metal strands 64 are left in that atmosphere for a predetermined amount of time. That is, the coating layer 42 of poly-para-xylylene resin is formed by the chemical vapour deposition of poly-para-xylylene, for example.

After the coating layer 42 of poly-para-xylylene has been formed on the metal strands 64, the wire core portion 62 is formed by bundling together those metal strands 64 into a stranded wire. The operation-use angle wire 60 is formed by coating the outer peripheral surface of the wire core portion 62 with the lubricant coating layer 66. As the lubricant coating layer 66, there is used a coating composition that includes at least one type selected from polytetrafluoroethylene (PTFE), carbon graphite and boron nitride, has epoxy resin and phenolic resin as a binder component, has a small percentage of an additive as a dispersant by weight, and has a solvent component that is an organic solvent such as methyl ethyl ketone or methyl isobutyl ketone. The coating composition is sprayed onto the wire core portion 62 on which the coating layer 42 of poly-para-xylylene resin has been formed, the solvent is allowed to dry naturally, and baking is performed at 190° C for 1 hour to form a coating layer (the lubricant coating layer 66) with a film thickness of 10 µm.

The operation-use angle wire 60 is formed by forming the coating layer 42 of poly-para-xylylene resin on the metal strands 64 and bundling together those metal strands 64 on which the coating layer 42 has been formed into a stranded wire, whereby the poly-para-xylylene resin covers the entire outer peripheral surfaces of the metal strands 64, and the adhesion between the poly-para-xylylene resin and the metal strands 64 is high, so oxidation and deterioration of the metal strands 64 can be controlled at the time of sterilization with high-temperature high-pressure steam. Further, the coating layer 42 of poly-para-xylylene resin has a low coefficient of friction, so frictional resistance at the time of operation of the endoscope is small.

Moreover, the operation-use angle wire 38 on which the coating layer 42 of poly-para-xylylene resin has been formed is coated with the lubricant coating layer 66 that reduces the coefficient of friction more than the coating layer 42 of poly-para-xylylene resin. Thus, a situation where the spiral tube 32 and the mesh tube 34 rub and wear due to friction of the operation-use angle wire 38 can be prevented or controlled.

It will be noted that, in the first to third embodiments, the coating layer 42 of poly-para-xylylene resin is formed separately on the mesh tube, the spiral tube and the operation-use angle wire, but the embodiments are not limited thereto. That is, it suffices for the coating layer 42 of poly-para-xylylene resin to be formed on at least one of the mesh tube, the spiral tube and the operation-use angle wire. The embodiment may also have a configuration where the coating layer 42 is formed on a combination of two or more of the mesh tube, the spiral tube and the operation-use angle wire or on all of the mesh tube, the spiral tube and the operation-use angle wire.

In the first embodiment, the coating layer 42 of poly-para-xylylene resin is formed on the metal strands that form the mesh tube, and thereafter the metal strands are braided together. However, the embodiment is not limited to this, and the coating layer 42 of poly-para-xylylene resin may also be formed on the mesh tube into which the metal strands have already been braided together.

In the third embodiment, the coating layer 42 of poly-para-xylylene resin is formed on the metal strands that form the operation-use angle wire, and thereafter the metal strands are bundled together into a stranded wire. However, the embodiment is not limited to this, and the coating layer 42 of poly-para-xylylene resin may also be formed on the outer peripheral surface of the operation-use angle wire that has already been formed by bundling together the metal strands into a stranded wire.

The shape of the spiral tube 32 and the braided shape of the mesh tube 34 in the endoscope flexible portion are not limited to the first to third embodiments, and may also have other configurations.

## Claims

1. An endoscope flexible portion (30) comprising:
a spiral tube (32) that is formed by spirally winding a strip-shaped member;
a mesh tube (34) that is disposed on the outer periphery of the spiral tube (32) and is formed by braiding together fine wires (34A);
an operation-use angle wire (38) that is disposed inside the spiral tube (32) and is formed by bundling together metal strands (64) into a stranded wire (62) in order to operate a curving portion disposed on a distal end side of the endoscope; and
an outer sheath member (36) that covers the outer periphery of the mesh tube (34),
wherein a coating layer (42) comprising poly-para-xylylene resin is formed on the outer peripheral surface of at least one of the spiral tube (32), the mesh tube (34) or the metal strands (64) forming the operation-use angle wire (38).

2. The endoscope flexible portion according to claim 1, wherein the mesh tube (34) is formed by forming the coating layer (42) comprising poly-para-xylylene resin on the fine wires (34A) and braiding together those fine wires (34A).

3. The endoscope flexible portion according to claim 1 or claim 2, wherein the operation-use angle wire (38) is formed by forming the coating layer (42) comprising poly-para-xylylene resin on the metal strands and bundling together those metal strands into a stranded wire.

4. The endoscope flexible portion according to claim 3, wherein the operation-use angle wire (38) on which the coating layer comprising poly-para-xylylene resin has been formed is coated with a lubricant (66) that reduces the coefficient of friction more than the coating layer (42) comprising poly-para-xylylene resin.

5. An endoscope (10) comprising an elongate insertion portion (12A) which is inserted inside a body cavity of a patient and which is formed by the endoscope flexible portion (30) according to any one of claim 1 to claim 4.
